Europäisches Patentamt

European Patent Office

Office européen des brevets

(19)

(11) Publication number: **0 121 317 B2**

## (12) NEW EUROPEAN PATENT SPECIFICATION

(45) Date of publication of the new patent specification : **27.11.91 Bulletin 91/48**

(51) Int. Cl.⁵ : **C12Q 1/28, // G01N33/72**

(21) Application number : **84301219.6**

(22) Date of filing : **24.02.84**

(54) **Peroxidase activity detection composition.**

(30) Priority : **01.03.83 US 471372**

(43) Date of publication of application :
**10.10.84 Bulletin 84/41**

(45) Publication of the grant of the patent :
**20.01.88 Bulletin 88/03**

(45) Mention of the opposition decision :
**27.11.91 Bulletin 91/48**

(84) Designated Contracting States :
**BE DE FR GB NL SE**

(56) References cited :
**US-A- 4 071 317**
**US-A- 4 071 318**
**US-A- 4 071 321**
**US-A- 4 251 222**
**US-A- 4 251 223**
**US-A- 4 386 053**
**CHEMICAL ABSTRACTS, vol. 97, part 21, 22nd November 1982, p. 450, no. 178369t, Columbus, Ohio (US; & JP-A-82 131 061**
**J. Clin. Path. (1955), 8, pages 249 - 251**
**J. Immunoassay (1981), 2(3&4) pages 187 - 204**
**J.A. Med. Assoc. (1934), 102, pages 1549 - 1550**
**Gradwohl's Clinical laboratory methods and diagnosis (1970), pages 1873 and 1874**

(56) References cited :
**Laboratoriums-Diagnostik (1953), Leipert, Piringer and Pilgerstorfer, page 417**
**HOPPE-SEYLER/THIERFELDER, Handbuch der physiologisch- und pathologisch-chemischen Analyse für Artze, Biologen und Chemiker (1953), 10th Edition, Volume 5, pages 407 - 408**
**Danish Medical Bulletin (1976), 23, No. 5/October, pages 230 - 235**
**Bergmeyer Methods of Enzymatic Analysis, 2nd English Edition, Volume 2, pages 685 - 690**
**J. Pharm. Sci. (1982), 71, no. 5, pages 585 - 587**
**ABS Abstract of DE-C-1771064**
**ABS Abstract of DE-C-2743548**
**Clinical Chemistry. Principles and Technics, 2nd Edition, Harper & Row, (1974), Chapter 23,"Hemoglobins, Myoglobins, and Haptoglobins"**

(73) Proprietor : **WARNER-LAMBERT COMPANY**
**201 Tabor Road**
**Morris Plains New Jersey 07950 (US)**

(72) Inventor : **Wells, Henry John**
**1145 Norwood Drive**
**Beaumont, TX 77706 (US)**

(74) Representative : **Jones, Michael Raymond et al**
**HASELTINE LAKE & CO. Hazlitt House 28**
**Southampton Buildings Chancery Lane**
**London WC2A 1AT (GB)**

EP 0 121 317 B2

**Description**

This invention relates to the use of a composition in the detection of peroxidase activity and in particular to the identification of peroxidase activity in various specimens. More particularly the invention discloses the preparation of a stable indicator solution useful for detection of peroxidase activity present in fecal occult blood, hemoglobin and biological fluids.

The detection of peroxidase activity has become an invaluable aid to the medical practitioner for the diagnosis of a number of disorders. Peroxidase activity has been found to be indicative of the presence of hemoglobin in a specimen and reported to be comparative with the heightened fertile period in females.

Over 100,000 persons in the United States are affected by cancer of the colon and rectum per year, occurring equally in both the male and female population. When the number of colorectal cancers occurring each year is combined with the number of cancers occurring in other digestive organs, including the oesophagus and stomach, such cancers of the digestive system account for more occurrences of cancer than any other single form of the disease. Contrary to many other forms of cancer, early diagnosis and treatment of digestive tract cancer does result in a cure rate of 80% to 90% of those persons affected by the disease. If, however, the disease is not detected until the later stages, the cure rate can drop drastically to 25% or less. Thus, early detection of the disease is critical to successful treatment of digestive tract cancer.

Most, but not all, cancers of the digestive tract bleed to a certain extent. Some blood found in the gastric contents and in vomitus is indicative of conditions associated with disorders of the mucous membrane, such as ulcers, diverticulitis, colitis, and carcinoma. In contrast, some blood is deposited on and in fecal matter excreted from the digestive system. The presence of blood in fecal matter is not normally detected, however, until gross bleeding occurs, resulting in blood visible to the naked eye. Gross bleeding, however, does not normally occur until the digestive tract cancers are in advanced stages.

It is known that digestive tract cancers in the early stages also tend to bleed, giving rise to occult (hidden) blood in fecal matter. Test equipment and test procedures have been developed for use by physicians in testing for the presence of occult blood in fecal matter. One of the most successful tests is manufactured and sold by Smith Kline Diagnostics of Sunnyvale, California, under the trademark "Hemoccult". The package for the "Hemoccult" test is disclosed in U.S. Patent No. 3,996,006 issued to J. F. Pagano.

Briefly, the Pagano test employs an absorbent white paper impregnated with a guaiac reagent and encased in a special test slide having openable flaps on both sides of the test slide. To use the Pagano test slide, one must obtain a sample of fecal matter, smear it onto the guaiac-impregnated paper by opening the panel on one side of the test slide, and thereafter close the panel. A panel on the opposite side of the test slide is then opened and a developing agent, which is a stabilized solution of hydrogen peroxide and denatured alcohol, is applied to the guaiac-impregnated paper. If occult blood is present in the fecal matter smeared on the opposite side of the paper, the product of the guaiac reaction will appear as a blue substance against the white paper background, providing a positive indication of the presence of blood in the fecal matter.

Although the Pagano test is excellent for use by physicians in their offices and by diagnostic laboratories, it is not the type of test that is readily adaptable for use by the ordinary person because of his adverse reaction to handling fecal matter and because of his lack of skill in interpreting the results. As stated above, the Pagano test requires that a specimen of fecal matter be obtained. Normally, a specimen is obtained by procuring a sample on the end of a spatula or a wooden depressor, which is then used to smear the specimen on the paper in the Pagano test slide.

Once the sample is obtained and the test procedure completed, both the test slide and the spatula or depressor must be disposed of. Disposal of the used materials can and does present a physical problem to, if not an adverse psychological reaction from, the ordinary person. Thus, the ordinary person is not likely to use the Pagano test because of its uncleanly nature and because of the disposal problems associated with the used test slide and spatula or depressor. Additionally, the ordinary person does not necessarily have the skill required to analyze, and thus form accurate conclusions from, the test results.

As an alternative, the ordinary person can initiate the Pagano test in his home and then forward the test slide to his physician or a laboratory for addition of the developing agent and analysis of the test. This procedure, however, requires cold storage of the test slide and specimen if there is a significant time lapse before the test can be completed. Certainly, the ordinary person does not wish to store a fecal speciment in his household refrigerator, normally the only cold storage available to him, until he can present the specimen to his physician or an appropriate laboratory. Thus, the general public is not likely to follow or comply with this alternative.

Another test for occult blood is suggested by D. E. Fonner in the U.S. Patent No. 2,838,377. The Fonner test, as disclosed, can be effected in a toilet bowl containing fecal matter. The basic test reagents employed by Fonner are o-tolidine and benzidine. These reagents in the presence of blood and other reactants produce a dye visible to the naked eye. Although the Fonner test appears to be a solution to the problem of finding a viable home test for occult blood, it has not met with success for two reasons. First, the above-listed reagents are in themselves known to cause cancer and these are not suitable for general public distribution. Additionally, the Fonner reagents have a relatively high rate of providing false indications of the presence of occult blood.

Thus, to date, the use of the Pagano test, the Fonner test, and other similar tests has been limited primarily to physicians and diagnostic laboratories. Although this limitation might not at first glance present a significant problem, it does limit the early detection of digestive tract cancers, primarily because patients will not see a physician until other symptoms of digestive tract cancers, such as gross bleeding, manifest themselves. Thus, early detection of cancer of the digestive tract still does not occur with the great majority of patients who contract the disease.

While the prior art has recognized the need for a simple and reliable test for detection of peroxidase activity, such test has not been available for routine home usage in an acceptable format.

A composition has been unexpectedly discovered which is useful for the detection of peroxidase activity.

According to a first aspect of the present invention there is provided a diagnostic kit for the simple, one-step detection in the home of peroxidase activity in a specimen, which avoids the manipulative handling of multiple reagents, characterised in that the diagnostic kit comprises a container containing a single, storage stable solution, the single solution containing at least 5% but not more than 45% by weight of water and comprising:

(a) an organic solvent;

(b) an indicator material that undergoes a characteristic colour change on oxidation within a given pH range;

(c) an oxidising agent capable of oxidising the indicator material in the presence of peroxidase activity; and

(d) a buffer to maintain the solution within said pH range;

said organic solvent being effective to stabilise the oxidising agent and being chosen from the following: alcohols, ethers, ketones, aromatic hydrocarbons, aliphatic hydrocarbons, chlorinated hydrocarbons, carboxylic acids, and mixtures thereof.

The solution comprises four essential ingredients which when admixed result in the formation of a stable optically visible indicator for the presence of peroxidase activity, such as the presence of hemoglobin in a particular specimen or sample. The solution in the presence of peroxidase activity, such as blood, develops a visble colour change. The solution is stable at room temperature and is easily used in an acceptable manner rendering it effective for home usage. The solution of the invention comprises four main ingredients:

a) a soluble indicator material;

b) an organic solvent for solubilizing the indicator;

c) an oxidizing agent capable of oxidizing the indicator material in the presence of peroxidase activity; and

d) a buffer.

According to a second aspect of this invention, there is provided a method for the detection of peroxidase activity, which comprises:

collecting a specimen of a material which is to be analyzed for the presence of peroxidase activity;

contacting the specimen with the single solution of the diagnostic kit according to a first aspect of the present invention; and

detecting the presence or absence of peroxidase activity.

The present invention is based on the detection of peroxidase activity present in hemoglobin and biological fluids. This peroxidase activity, also referred to as catalytically active substances, in the case of blood are identified in hemoglobin. These substances belong to the general class of hemoproteins, conjugate proteins all of which have the same prosthetic group, iron protoporphyrin or haem. This prosthetic group has the ability to catalyze the transfer of oxygen form an oxygen source to an acceptor which in turn becomes oxidized. The acceptor is a colourless precursor until it becomes oxidized wherein the oxidized form indicates the presence of the peroxidase activity by colour formation.

The solution used in this invention contains an indicator material previously solubilized in an organic solvent plus an oxygen source, notably an oxidizing agent. In the presence of peroxidase activity, such as hemoglobin, which contains the catalytic active group, the indicator is oxidized and coloured.

The instant invention represents a significant advance in the art by the preparation of a stable diagnostic composition which contains all of the essential materials necessary for the detection of peroxidase activity. This composition is used by merely contacting the specimen to be analyzed with the solution in one simple step and avoids the prior art manipulative handling of multiple reagents and specimens.

Any indicator material may be useful in the practice of this invention which is capable of accepting oxygen and being oxidized to a coloured dye in the presence of the oxygen source. Exemplary indicator material include guaiac, aniline and its derivatives, o-tolidine, o-toluidine, p-toluidine, benzidine, tetramethylbenzidine, di-anisidine, o-cresol, m-cresol, alphanophthol, beta-naphthol, catechol, guaiacol, pyrogallol and mixtures thereof. The preferred indicator material is guaiac because of its recognition as an effective indicator, its noncarcinogenicity and commercial availability. In the presence of an oxygen source and peroxidase activity, guaiac changes from colourless to a blue colour.

The amount of indicator employed in the formulation of the invention is dependent upon the sensitivity desired, that is the extent of colour change evidenced from the oxidation reaction. Preferred useful amounts may range from about 0.01% to about 3.0% and preferably about 0.25% to about 2.0% by weight, based upon the weight of the entire formulation. Amounts above about 3% are not desired since they may result in hypersensitivity showing false positive results. Amounts below about 0.01% are not recommended since they do not provide sufficient colour development for accurate detection of peroxidase activity.

An organic solvent is present in the formulations of this invention to stabilize the oxidising agent present to prevent premature oxidation of the indicator material. A wide range of organic solvents may be used which are nonreactive with the formulation ingredients and which are preferably miscible in water.

Representative organic solvents include compounds selected from 1) alcohols, such as methanol, ethanol, propanol, butanol, isopropanol and isobutanol; 2) ethers, such as diethylether, diisopropylether; 3) ketones, such as acetone, methylethyl ketone, methylisobutylketone; 4) aromatic hydrocarbons such as benzene and toluene; 5) chlorinated hydrocarbons such as methylenechloride, chloroform, carbontetrachloride; 6) carboxylic acids, such as acetic acid and propionic acid, and mixtures thereof. Most preferred solvents are selected from the group consisting of methanol, ethanol, isopropanol and mixtures thereof.

The amount of solvent employed is that amount which is sufficient to stabilize the oxidizing agent. This amount may vary widely and preferable ranges in amounts up to 75% by weight of the total formulation. The exact amount of solvent for a particular formulation can be readily determined since it is that amount which is added to the remaining ingredients to yield a 100% solution.

The oxidizing agent is selected from hydrogen peroxide or materials that will yield hydrogen peroxide in the presence of water. The hydrogen peroxide is the oxidizing agent that promotes the reaction between the indicator and the peroxidase activator to produce the indicator's colour change. The oxidizing agent must be capable of being combined with the indicator only in the presence of the peroxidase activity material and remain stable in the formulation during storage. It should be recognized that oxidation of the indicator must be limited in the presence of the oxidizing agent because of the inherent presence of water in the formulation. This inhibition is achieved by use of the organic solvents described above.

Representative oxidizing agents include both organic and inorganic peroxides. Illustrative compounds include hydrogen peroxide, cumene hydroperoxide, tertiary butyl hydroperoxide and mixtures thereof.

The oxidizing agent is employed in amounts of about 0.15% to about 3.0% by weight, based on the weight of formulation, and preferably from about 1.0% to 3.0% by weight. Higher amounts are not preferred since they may result in premature oxidation of the indicator and possible precipitation of ingredients during storage whereas lower amounts will not enable sufficient oxidation for visual detection, possible slow reactivity and loss of peroxidase activity.

A buffer is employed in the formulation to aid in stability and to provide the optimum pH value to enable catalytic activity to occur. It is essential that the buffer selected maintain the pH within a range in which the indicator material changes colour upon oxidation. Normally this will be between a pH of about 2 and about 8 and preferably between about 3 and about 5. Higher pH values should be avoided to prevent autoxidation of the indicator resulting in false positive results. Lower pH values do not result in efficient oxidation.

Representative buffers include citrate, tartrate, phosphate, acetate and mixtures thereof with citrate being preferred.

The composition of the invention may be prepared by routine procedures. The exact manner of mixing the reagents is not critical except that the indicator should be solubilised prior to the addition of the remaining ingredients. The order of addition thus will be dependent upon the solubility of the particular indicator. For example, when using guaiac as the indicator material it is preferably solubilised in the organic solvent, filtered to remove undissolved particles, mixed with the oxidizing agent which is previously solubilised in water and finally admixed with the buffer solution to adjust the pH value. In contrast, when using water soluble indicators, such as benzidine or tolidine, such indicators are dissolved in water and mixed with the organic solvent or initially mixed with an aqueous alcohol which contains the requisite water and organic solvent concentration. The buffer is then added to adjust the pH value and the solution stored. Any suitable temperature for performing the mixing procedure is employed with room temperature preferred, that is 20°C to 25°C.

When using water during processing it is important to maintain the final water content below about 45% by weight. Amounts of water from about 5 to about 45% can be tolerated in the system. Higher amounts should be avoided since such amounts may cause premature decomposition of the oxidizing agent.

As will be readily recognized by one of ordinary skill, the present invention represents a significant advance of the prior art diagnostic aids employing reagent impregnated papers. All that need be done with the single composition system defined herein, is to place the compoistion onto the contents to be tested for peroxidase activity and to observe the composition for the characteristic colour change. The diagnostic kit and method for determining the presence of hemoglobin, such as in occult blood, through peroxidase activity in accordance with the present invention does not require the conventional handling of specimen, such as feces and can be simply performed in the home without need for professional interpretation of results.

In accordance with the present invention, a single container is employed containing all the required test reagents, which when placed in contact with the specimen result in a characteristic colour change resulting from the indicator oxidation reaction. This container is thus useful in a diagnostic kit for the detection of peroxidase activity.

The reagents may be dispensed in any convenient manner. Conventional dispensing means can include dropper bottle, spray delivery systems and aerosol delivery systems. Alternatively, conventional thickening additives may be employed in sufficient quantities to enhance thixotrophy of the solution. Such solution can then be dispensed by roller means, dropper means and/or conventional physical manipulation. Such thixotrophy additives may include but are not limited to silica gels, polyethylene glycol, methylcellulose, polyvinyl alcohol, poly (ethylene oxide) and quaternary ammonium derivatives.

The specimen may be obtained by routine correction procedures. Without being limited thereto, such procedures include standard techniques for isolating fuel occult blood, hemoglobin, biological fluids and so forth. The term biological fluids as used herein includes saliva, urine, gastric fluids, vaginal secretions and cervical secretions. Such specimens may be obtained using standard absorptive materials including paper strips and wood products.

The following examples are given to illustrate the invention, but are not deemed to be limiting thereof. All percentages given throughout the specification are based upon weight unless otherwise indicated.

Example I (Run 1)

This example demonstrates the preparation of compositions according to the invention using organic soluble indicators.

To a flask was added 100 cc of methanol followed by the addition of 1 gram of guaiac powder. The contents were mixed for approximately 30 minutes to dissolve the guaiac indicator in the methanol solvent. The solution was filtered to remove undissolved particles.

Fifty (50) cc of the resulting solution were mixed with 4.5 cc of 30% hydrogen peroxide followed by the addition of 15 cc of 0.1 M citrate buffer to adjust the pH to 5.0. Ten (10) cc of water were added to prevent crystallization of buffer followed by increasing the volume to 100 cc by the addition of fresh methanol. The resulting solution exhibited a slight amber colour.

The effectiveness of this solution for detecting peroxidase activity was performed by contacting previously prepared hemoglobin specimens with this solution. The hemoglobin specimens were prepared by taking diluted blood samples, drops of which were placed onto Whatman No. 1 filter paper. A drop of the invention's formulation was applied to the diluted blood sample and a distinct change of colour from colourless to blue was observed. The hemoglobin test procedure has been repeated using the aforementioned solution after storage at elevated temperatures (45°C) for 6 weeks. No appreciable loss of reactivity was observed.

To determine stability under accelerated shelf-life conditions, the concentration of hydrogen peroxide was determined at various time intervals. The results indicate a minor drop in hydrogen peroxide content indicating high stability of this formulation.

Example II (Runs 2 to 5)

The procedure of Example I was repeated using different organic solvents with the guaiac indicator.
Run 2 used a 1% ethanol solution.
Run 3 used a 3% toluene solution.
Run 4 used a 3% chloroform solution.
Run 5 used a 3% acetone solution.
Runs 2 and 5 resulted in the formation of a single phase solution and as in Run 1 had an amber colour. Runs 3 and 4 exhibited a two-phase (organic-aqueous) system, both phases being effective in the detection of peroxidase activity.

The solutions prepared in runs 2 through 5 were used in the detection of hemoglobin by the procedure of Example I. All solutions resulted in a positive colour change. Only Run 2 using ethanol as solvent was placed under accelerated storage conditions. This solution exhibited the same stability as observed in Run 1.

Example III (Run 6 and 7)

This example demonstrates the use of water soluble indicators within the scope of this invention.

To a flask was added 100 cc of water followed by the addition of 1 gram of indicator. The contents were mixed for approximately 30 minutes to dissolve the indicator in the water. The solution was filtered to remove all undissolved particles. In Run 6 the indicator was o-tolidine whereas in Run 7 the indicator was tetramethyl-benzidine. Twenty (20) cc of the resultant solutions were mixed with 4.5 ml of 30% hydrogen peroxide followed by the addition of 15 ml of 0.1 M citrate buffer to adjust the pH to 5.0. Sixty (60) cc of ethanol were then added to bring the final volume to 100 cc. The resulting solutions both exhibited a red-orange colour.

The effectiveness of the solutions for detecting peroxidase activity was performed by contacting previously prepared hemoglobin specimens with this solution. The hemoglobin specimens were prepared by taking diluted blood samples, drops of which were placed onto Whatman No. 1 filter paper. A drop of this invention's formulation was applied to the diluted blood sample and a distinct change of colour to a blue-green was observed for both solutions.

Example IV (Runs 8 to 11)

This example demonstrates the effectiveness of the compositions of this invention using various concentrations of guaiac.

The procedure of Example I was repeated except that methanol was replaced by ethanol as the organic solvent. The concentration of guaiac employed and the results obtained are set forth in Table I. The results indicate excellent stability using a wide range of guaiac concentrations.

Whatman is a registered trade mark.

TABLE I

| Run No. | Guaiac concentrations (%) | Reactivity to .25 mcg hemoglobin/ml of solution | Stability studies |
|---|---|---|---|
| 8 | 3.0 | + | Retains sensitivity after 6 weeks at 45°C |
| 9 | 1.5 | + | Retains sensitivity after 6 weeks at 45°C |
| 10 | 0.75 | + | Retains sensitivity after 6 weeks at 45°C |
| 11 | 0.50 | + | Retains sensitivity after 6 weeks at 45°C |

Example V (Run 12 and 13)

This example demonstrates the use of various thickening agents in the compositions of this invention.

The procedure of Example 1 was repeated except that methanol was replaced by isopropanol as the organic solvent. The thickener as set forth in Table II was added to a previously prepared solution and admixed for approximately 5 minutes to obtain a uniform mixture. The additives used were as follows:

Run 12 PEG-4000: polyethylene glycol, gram Molecular Weight 4000.

Run 13 Poly Ox®: product of Union Carbide Co. Used as a 0.25% solution in isopropyl alcohol.

The viscosity of the resulting formulations were higher than in the absence of these thickening agents. These high viscosity compositions exhibited a thick-flowable formulation.

The effectiveness of these thickened solutions for detecting peroxidase activity was determined by contracting previously prepared hemoglobin with these solutions. The hemoglobin specimens were prepared by taking diluted blood samples, drops of which were placed onto Whatman No. 1 filter paper. A drop of the inventive formulations was applied to the diluted blood sample and a distinct change from colorless to a blue color was observed. Results from stability studies are set forth in Table II.

TABLE II

| Run No. | 3% Guaiac solution (ml) | Hydrogen peroxide (ml) | Citrate buffer (ml) | Additive 1 | Reactivity to .25 mcg hemoglobin per ml | Stability |
|---|---|---|---|---|---|---|
| 12 | 42 | 6 | 10 | PEG-4000 | + | 5 weeks at RT |
| 13 | 40 | 4 | 20 | Poly Ox® | + | 6 weeks at 60°C |

**Claims**

1. A diagnostic kit for the simple, one-step detection in the home of peroxidase activity in a specimen, which avoids the manipulative handling of multiple reagents, characterised in that the diagnostic kit comprises a container containing a single, storage stable solution, the single solution containing at least 5% but not more than 45% by weight of water and comprising:
(a) an organic solvent;
(b) an indicator material that undergoes a characteristic colour change on oxidation within a given pH range;
(c) an oxidising agent capable of oxidising the indicator material in the presence of peroxidase activity; and
(d) a buffer to maintain the solution within said pH range;
said organic solvent being effective to stabilise the oxidising agent and being chosen from the following: alcohols, ethers, ketones, aromatic hydrocarbons, aliphatic hydrocarbons, chlorinated hydrocarbons, carboxylic acids, and mixtures thereof.

2. A diagnostic kit according to claim 1, wherein the organic solvent is methanol, ethanol, isopropanol or a mixture thereof.

3. A diagnostic kit according to claim 1 or 2, wherein: the indicator material is present in an amount of from 0.01 to 3.0%; the oxidising agent is present in an amount of from 0.15 to 3.0% by weight; the buffer is present in an amount sufficient to maintain the pH of the composition within the range from 2.0 to 8.0, all weights being based on the weight of the solution.

4. A diagnostic kit according to claim 3, wherein the indicator material is present in an amount of from 0.25 to 2.5% by weight; and the oxidising agent is present in an amount of from 1.0 to 3.0% by weight.

5. A diagnostic kit according to any preceding claim, wherein the organic solvent is present in an amount of up to 75% by weight of the final composition.

6. A diagnostic kit according to any preceding claim, wherein the soluble indicator material is chosen from the following: guaiac, aniline and its derivatives, o-tolidine, o-toluidine, p-toluidine, benzidine, tetramethyl-benzidine, di-anisidine, o-cresol, m-cresol, p-cresol, alpha-naphthol, beta-naphthol, catechol, guaiacol, pyrogallol and mixtures thereof.

7. A diagnostic kit according to any preceding claim, wherein the oxidising agent is chosen from the following: organic peroxides, inorganic peroxides and mixtures thereof; preferably from hydrogen peroxide, cumene hydroperoxide, tertiary butyl hydroperoxide and mixtures thereof.

8. A diagnostic kit according to any preceding claim, to be used in the detection of haemoglobin.

9. A diagnostic kit according to any preceding claim further comprising a thickener.

10. A diagnostic kit according to claim 9, wherein the thickener is a polyethylene glycol, preferably having a gram molecular weight of from 4000, or Poly Ox[R].

11. A diagnostic kit for the simple, one-step detection in the home of occult blood in faecal matter, which avoids the manipulative handling of multiple reagents, characterised in that the diagnostic kit comprises a closed container containing a single, storage stable solution which comprises:
(a) guaiac in an amount of from 0.01% to 3.0% by weight of the final solution;
(b) hydrogen peroxide in an amount of from 0.15% to 3.0% by weight of the final solution;
(c) a sufficient amount of a buffer to maintain the pH of the solution in the range from 2.0 to 8.0; and
(d) an organic solvent, which is non-reactive with the other components of the solution, in an amount sufficient to stabilise the oxidising agent, the organic solvent being chosen from methanol, ethanol, isopropanol and mixtures thereof and containing not more than 45% water.

12. A diagnostic kit according to any preceding claim, further comprising a specimen collection means.

EP 0 121 317 B2

13. A method for the testing of peroxidase activity, which comprises: collecting a specimen of a material which is to be analysed for the presence of peroxidase activity; contacting the specimen with the single solution of the diagnostic kit according to any preceding claim; and detecting the presence or absence of peroxidase activity.

14. A method according to claim 13, wherein the specimen is faecal matter and the detection of peroxidase activity is evidence of faecal occult blood.

15. A method according to claim 13, wherein the specimen is a biological fluid which may be chosen from saliva, urine, gastric fluids, vaginal secretions and cervical secretions.

**Patentansprüche**

1. Diagnosebesteck, mit dessen Hilfe zuhause ein einfacher, einstufiger Nachweis einer Peroxidaseaktivität in einer Probe möglich ist, ohne daß man sich mehrerer Reagenzien bedienen muß, dadurch **gekennzeichnet**, daß das Diagnosebesteck aus einem Behälter mit einer einzigen, lagerungsstabilen Lösung mit mindestens 5 Gew.-%, jedoch nicht mehr als 45 Gew.-% Wasser und

(a) einem organischen Lösungsmittel;

(b) einem Indikatormaterial, das bei der Oxidation innerhalb eines gegebenen pH-Bereichs eine charakteristische Farbänderung erfährt;

(c) einem zur Oxidation des Indikatormaterials in Gegenwart von Peroxidaseaktivität fähigen Oxidationsmittel und

(d) einem Puffer, der die Lösung innerhalb des besagten pH-Bereichs hält,

wobei das organische Lösungsmittel eine Stabilisierung des Oxidationsmittels bewirkt und aus Alkoholen, Ethern, Ketonen, aromatischen Kohlenwasserstoffen, aliphatischen Kohlenwasserstoffen, chlorierten Kohlenwasserstoffen, Carbonsäuren und Mischungen derselben ausgewählt ist, besteht.

2. Diagnosebesteck nach Anspruch 1, dadurch **gekennzeichnet**, daß das organische Lösungsmittel aus Methanol, Ethanol, Isopropanol oder einem Gemisch derselben besteht.

3. Diagnosebesteck nach Anspruch 1 oder 2, dadurch **gekennzeichnet**, daß es, bezogen auf das Gewicht der Lösung, 0,01 bis 3,0 Gew.-% Indikatormaterial und 0,15 bis 3,0 Gew.-% Oxidationsmittel und eine zur Aufrechterhaltung des pH-Werts der Masse in einem Bereich von 2,0 bis 8,0 ausreichende Menge Puffer enthält.

4. Diagnosebesteck nach Anspruch 3, dadurch **gekennzeichnet**, daß das Indikatormaterial in einer Menge von 0,25 bis 2,5 Gew.-% und das Oxidationsmittel in einer Menge von 1,0 bis 3,0 Gew.-% vorhanden sind.

5. Diagnosebesteck nach einem der vorhergehenden Ansprüche dadurch **gekennzeichnet**, daß das organische Lösungsmittel in einer Menge von bis zu 75 Gew.-% der fertigen Masse vorhanden ist.

6. Diagnosebesteck nach einem der vorhergehenden Ansprüche, dadurch **gekennzeichnet**, daß das lösliche Indikatormaterial aus Guaiacöl, Anilin und seinen Derivaten, o-To lidin, o-Toluidin, p-Toluidin, Benzidin, Tetramethylbenzidin, Dianisidin, o-Cresol, m-Cresol, p-Cresol, alpha-Naphtol, beta-Naphtol, Brenzcatechin, Guaiacol, Pyrogallol und Mischungen derselben ausgewählt ist.

7. Diagnosebesteck nach einem der vorhergehenden Ansprüche, dadurch **gekennzeichnet**, daß das Oxidationsmittel aus organischen Peroxiden, anorganischen Peroxiden und Mischungen derselben, vorzugsweise Wasserstoffperoxid, Cumolhydroperoxid, tert.-Butylhydroperoxid und Mischungen derselben, ausgewählt ist.

8. Diagnosebesteck nach einem der vorhergehenden Ansprüche zur Verwendung beim Nachweis von Hämoglobin.

9. Diagnosebesteck nach einem der vorhergehenden Ansprüche, das zusätzlich ein Dickungsmittel enthält.

10. Diagnosebesteck nach Anspruch 9, dadurch **gekennzeichnet**, daß das Dickungsmittel aus einem Polyethylenglycol, vorzugsweise einem solchen mit einem Grammolekulargewicht von 4000, oder Poly Ox$^R$ besteht.

11. Diagnosebesteck, mit dessen Hilfe zuhause ein einfacher, einstufiger Nachweis von Okkultblut im Stuhl möglich ist, ohne daß man sich mehrerer Reagenzien bedienen muß, dadurch **gekennzeichnet**, daß es aus einem geschlossenen Behälter mit einer einzigen, lagerungsstabilen Lösung, enthaltend

(a) 0,01 bis 3,0 Gew.-%, bezogen auf die endgültige Lösung, Guaiacöl;

(b) 0,15 bis 3,0 Gew.-%, bezogen auf die fertige Lösung, Wasserstoffperoxid;

(c) eine zur Aufrechterhaltung des pH-Werts der Lösung im Bereich von 2,0 bis 8,0 ausreichende Menge eines Puffers und

(d) ein mit den anderen Bestandteilen der Lösung nicht reaktionsfähiges und nicht mehr als 45% Wasser enthaltendes organisches Lösungsmittel in Form von Methanol, Ethanol, Isopropanol und Mischungen derselben in einer zur Stabilisierung des Oxidationsmittels ausreichenden Menge, besteht.

12. Diagnosebesteck nach einem der vorhergehenden Ansprüche, dadurch **gekennzeichnet**, daß es

8

zusätzlich eine Probensammeleinrichtung enthält.

13. Verfahren zum Nachweis von Peroxidaseaktivität, dadurch **gekennzeichnet,** daß man eine Probe eines auf die Anwesenheit von Peroxidaseaktivität zu analysierenden Materials sammelt, die Probe mit der einzigen Lösung des Diagnosebestecks nach einem der vorhergehenden Ansprüche in Berührung bringt und die Anwesenheit oder Abwesenheit von Peroxidaseaktivität bestimmt.

14. Verfahren nach Anspruch 13, dadurch **gekennzeichnet,** daß es sich bei der Probe um Fäkalmaterial handelt und der Nachweis von Peroxidaseaktivität als Beleg für fäkales okkultes Blut dient.

15. Verfahren nach Anspruch 13, dadurch **gekennzeichnet,** daß es sich bei der Probe um eine biologische Flüssigkeit, ausgewählt aus Speichel, Urin, Magensaft, Vaginalsekret und Zervikalsekret, handelt.

## Revendications

1. Un kit de diagnostic pour la détection simple, en une seule étape et chez soi, de l'activité peroxydasique dans un échantillon, qui permet d'éviter la manipulation de nombreux réactifs, caractérisé en ce que le kit de diagnostic comprend un récipient contenant une solution, stable au stockage et unique, contenant au moins 5% et pas plus de 45% en poids d'eau et comprenant:

(a) un solvant organique;

(b) un indicateur qui subit un changement de coloration caractéristique lors de l'oxydation dans un domaine de pH donné;

(c) un agent d'oxydation capable d'oxyder cet indicateur en présence d'une activité peroxydasique; et

(d) un tampon pour maintenir la solution dans ce domaine de PH,

ledit solvant organique étant efficace pour stabiliser l'agent d'oxydation et étant choisi dans le groupe comprenant les alcools, éthers, cétones, hydrocarbures aromatiques, hydrocarbures aliphatiques, hydrocarbures chlorés, acides carboxyliques et leurs mélanges.

2. Un kit de diagnostic selon la revendication 1, dans lequel le solvant organique consiste en méthanol, éthanol, isopropanol ou un de leurs mélanges.

3. Un kit de diagnostic selon la revendication 1 ou la revendication 2, dans lequel l'indicateur est présent en une proportion de l'ordre de 0,01 à 3,0%; l'agent d'oxydation est présent en une proportion comprise entre 0,15 et 3,0% en poids; le tampon est présent en une quantité suffisante pour maintenir le pH de la composition dans le domaine de pH compris entre 2,0 et 8,0, tous les poids étant exprimés par rapport au poids de ladite solution.

4. Un kit de diagnostic selon la revendication 3, dans lequel l'indicateur est présent en une proportion comprise entre 0,25 et 2,5% en poids, et l'agent d'oxydation est présent en une proportion comprise entre 1,0 et 3,0% en poids.

5. Un kit de diagnostic selon une quelconque des revendications précédentes, dans lequel le solvant organique est présent en une quantité pouvant atteindre 75% en poids de la composition finale.

6. Un kit de diagnostic selon une quelconque des revendications précédentes, dans lequel l'indicateur soluble est choisi parmi les suivants: gayac, aniline et ses dérivés, o-tolidine, o-toluidine, p-toluidine, benzidine, tétraméthylbenzidine, di-anisidine, o-crésol, m-crésol, p-crésol, alphanaphtol, béta-naphtol, catéchol, gayacol, pyrogallol et leurs mélanges.

7. Un kit de diagnostic selon une quelconque des revendications précédentes, dans lequel l'agent d'oxydation est choisi parmi les suivants: peroxydes organiques, peroxydes minéraux et leurs mélanges; et de préférence parmi peroxyde d'hydrogène, hydroperoxyde de cumène, hydroperoxyde de tertiobutyle et leurs mélanges.

8. Un kit de diagnostic selon une quelconque des revendications précédentes, à utiliser pour la détection d'hémoglobine.

9. Un kit de diagnostic selon une quelconque des revendications précédentes, caractérisé en ce qu'il comprend en outre un épaississeur.

10. Un kit de diagnostic selon la revendication 9, dans lequel l'épaississeur consiste en un polyéthylèneglycol présentant de préférence un poids moléculaire en gramme de l'ordre de 4 000 ou en Poly Ox®.

11. Un kit de diagnostic pour la détection simple, en une seule étape et chez soi, du sang occulté dans les matières fécales, qui permet d'éviter la manipulation de nombreux réactifs, caractérisé en ce que le kit de diagnostic comprend un récipient clos contenant une solution stable au stockage et unique qui comprend:

(a) du gayac en une proportion comprise entre 0,01% et 3,0% par rapport au poids de la solution finale;

(b) du peroxyde d'hydrogène en une proportion comprise entre 0,15% et 3,0% par rapport au poids de la solution finale;

(c) une quantité suffisante de tampon pour maintenir le pH de la solution dans des limites de 2,0 à 8,0; et

(d) un solvant organique ne réagissant pas avec les autres composants de la solution, en une quantité suffisante pour stabiliser l'agent d'oxydation, ce solvant organique étant choisi parmi les suivants: méthanol, éthanol, isopropanol et leurs mélanges, et en ce qu'il contient en outre pas plus de 45% d'eau.

12. Un kit de diagnostic selon une quelconque des revendications précédentes, caractérisé en ce qu'il comprend en outre des moyens de collecte de l'échantillon.

13. Un procédé de détection de l'activité peroxydasique qui consiste: à recueillir un spécimen d'un produit dans lequel on doit analyser la présence d'une activité peroxydasique; à mettre cet échantillon au contact de la solution unique du kit de diagnostic telle que décrite dans l'une quelconque des revendications précédentes; et à détecter la présence ou l'absence d'une activité peroxydasique.

14. Un procédé selon la revendication 13, dans lequel le spécimen est une matière fécale et la détection d'une activité peroxydasique représente une preuve de la présence de sang fécal occulté.

15. Un procédé selon la revendication 13, dans lequel le spécimen est un fluide biologique pouvant être choisi parmi les suivants: salive, urine, fluides gastriques, sécrétions vaginales et sécrétions cervicales.